(19) Europäisches Patentamt
European Patent Office
Office européen des brevets

(11) Publication number: **0 270 295 A2**

(12) # EUROPEAN PATENT APPLICATION

(21) Application number: 87310377.4

(51) Int. Cl.4: A61K 39/385

(22) Date of filing: 25.11.87

(30) Priority: 03.12.86 US 937508

(43) Date of publication of application: 08.06.88 Bulletin 88/23

(84) Designated Contracting States: AT BE CH DE ES FR GB GR IT LI LU NL SE

(71) Applicant: CONNAUGHT LABORATORIES LIMITED
1755 Steeles Avenue West
Willowdale Ontario M2N 5T8(CA)

(72) Inventor: Brown, Cynthia S.
411 1/2 Main Street, Apt. 204
Stroudsburg Pennsylvania 18360(US)
Inventor: Hamel, Coleman R.
1407 West Union Blvd.
Bethlehem Pennsylvania 18018(US)
Inventor: Lemmon, Robert D.
7311 Jasmine Drive
Hanover Park Illinois 60103(US)
Inventor: McVerry, Patrick H.
Norton Road
Stroudsburg Pennsylvania 18360(US)

(74) Representative: Burford, Anthony Frederick et al
W.H. Beck, Greener & Co. 7 Stone Buildings
Lincoln's Inn
London WC2A 3SZ(GB)

(54) **Conjugate vaccine.**

(57) The immune response to the weakly-immunogenic hemagglutinin subunit protein antigen (HA) of influenza virus, type A or B, and other similar weakly-immunogenic viral protein antigens, is significantly increased by covalently linking the HA to a highly immunogenic protein, such as that of a bacterial organism, for example, diphtheria toxoid and tetanus toxoid.

EP 0 270 295 A2

Xerox Copy Centre

## CONJUGATE VACCINE

The present invention relates to certain novel vaccines comprising conjugates of weakly-immunogenic viral proteins and highly immunogenic proteins.

Whole virus vaccines administered to the body elicit an immune response by the formation of antibodies to the viral antigen. In the case of the influenza virus, it is known that the surface antigens, namely hemagglutinin (HA) and neuraminide (NA), are the main antibody-inducing antigens.

The trend with respect to vaccines is away from whole virus vaccines and towards more purified materials. The influenza surface antigens have been isolated and have been employed in vaccines. However, such purified protein vaccines are only weakly immunogenic and are incapable of inducing a sufficiently high immune response to be effective in many classes of individuals.

In accordance with the present invention, a novel conjugate is provided in which a weakly-immunogenic viral protein antigen is covalently bonded to a strongly immunogenic protein. By covalently bonding the viral protein antigen to the strongly immunogenic protein, a significantly enhanced immune response to the normally weakly immunogenic antigen can be elicited from a vertebrate to which the conjugate is administered.

The invention has particular application to the hemagglutinin glycoprotein of a variety of strains of the influenza virus, including type A and type B influenza, but also is applicable to the potentiation of other viral protein antigens, including rabies glycoprotein g, polio virus glycoproteins and rotavirus glycoproteins.

In addition to such protein antigens isolated from natural sources, included within the scope of the invention are genetically-engineered peptides and synthetically-produced peptides corresponding to the viral protein antigens.

In forming the conjugates, the hemagglutinin trimer first is removed from the whole virus by any convenient method, for example, by conventional bromelain cleavage. In this way, the hydrophilic portions of the hemagglutinin are separated from the hydrophobic portion, which remains with the viral core.

The immunogenicity of the HA-subunit is considerably less than that of the whole virus. In accordance with this invention, the immunogenicity is increased significantly by covalently bonding the HA-trimer to a highly immunogenic protein.

The highly immunogenic protein is one capable of eliciting a humoral antibody and/or a cell mediated immune response. By carrying the purified HA protein to the response site, the highly immunogenic protein potentiates the immune response of the normally weakly-immunogenic HA-subunit protein. An increased specific T-cell proliferation is observed as is a specific humoral response.

The highly immunogenic protein may be a bacterial protein, such as bacterial exotoxoid, preferably diphtheria toxoid or tetanus toxoid. Other proteinaceous materials which may be used include antibody molecules, fimbrial proteins and bacterial outer membrane proteins.

It has previously been suggested to covalently link molecules for a variety of purposes. For example, U.S. Patents Nos. 4,496,538 and 4,619,828 to Gordon describe the covalent linking of the capsular polysaccharide from Haemophilus influenzae b to diphtheria or tetanus toxoid to elicit T-cell dependent responses to the polysaccharide. A similar response was observed by Cryz et al with a Pseudomonas aeruginosa polysaccharide-tetanus toxoid conjugate vaccine, as reported in The Journal of Infectious Diseases, Vol. 154 No. 4, October 1986, pp. 682-688.

In U.S. Patent No. 4,565,696 to Heath et al, the immune response of a peptide or protein immunogen is potentiated by covalent linking to vesicle surfaces above a minimum ratio of protein to lipid. A similar disclosure appears in published international patent application WO 83/02069 published June 23, 1983.

Lee et al in Molecular Immunology, Vol. 17, pp. 749 to 756 (1980) describe the covalent bonding of the beta subunit of human chorionic gonadotropin (hCG) to tetanus toxoid and the use of the conjugate to obtain significant levels of antibodies to hCG peptides.

Petrov et al in Reports of the Academy of Sciences of the USSR, 1984, Vol. 277 No. 3, pp. 752 to 755 reported an enhanced immune response from both HA and NA influenza virus subunits by covalent bonding.

As far as the applicants are aware, however, there has been no suggestion in the prior art to provide a conjugate suitable for use as a vaccine wherein a normally weakly-immunogenic viral protein antigen is covalently bonded to a strongly immunogenic protein to obtain an enhanced immunogenicity to the viral protein antigen. In addition, there is no indication in the prior art which would reasonably predict that the enhanced immunogenicity would result from conjugation of the viral protein antigen to the strongly immunogenic protein.

In preparing the conjugate of the invention, it is necessary to link the two protein molecules covalently.

Any suitable conjugation procedure may be employed. Preferably, the conjugation chemistry should be such as to minimize the formation of unwanted side products, such as homoconjugates or polymers, while maximizing the yield of functionally-active conjugate.

Heterobifunctional cross-linkers of the maleimide-N-hydroxy-succinimide ester type are preferred, since they provide the desired chemistry. The sequential reaction of the N-hydroxy-succinimide ester with amino groups on one protein, followed by reaction of the maleimide component with free sulfhydryl groups introduced to or present in the other protein, yields conjugates which are free from the polymeric products that arise from the use of homobifunctional cross-linkers, such as glutaraldehyde. It is preferred to introduce the sulfhydryl group to the weakly-immunogenic protein and react the maleimide-N-hydroxy succinimide ester to the strongly-immunogenic carrier protein.

The reactions which are involved may be depicted as follows:

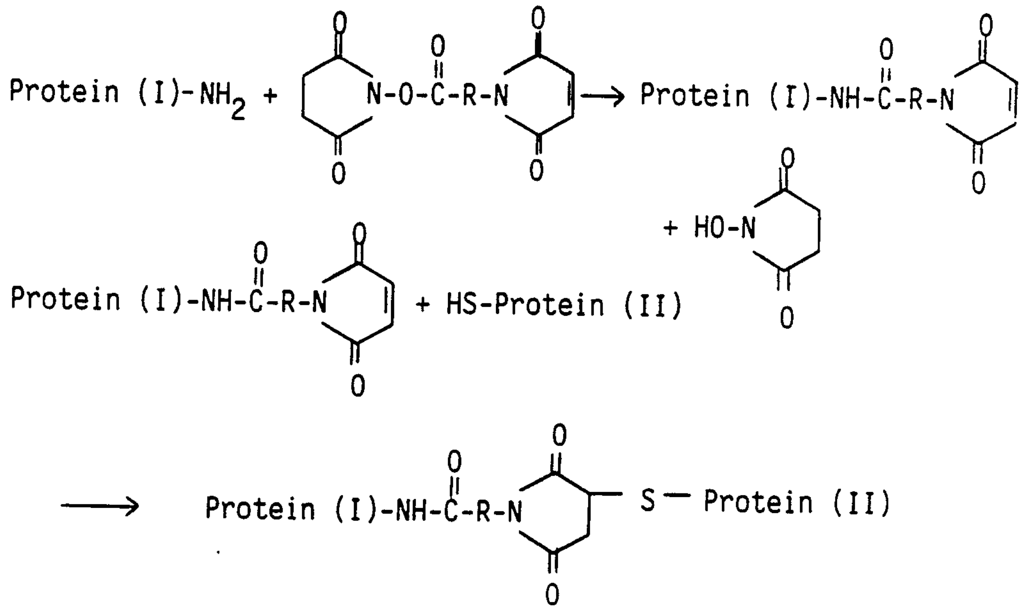

The group R in the maleimide-N-hydroxysuccinimide ester may be any convenient acid residue, and may include an optionally-substituted phenylene group, cycloalkylene group or alkylene group containing from 1 to 12 carbon atoms, preferably 5 to 12 carbon atoms. Examples of such bifunctional esters include maleimido-caproic-N-hydroxysuccinimide ester (MCS), maleimido-benzoyl-N-hydroxysuccinimide ester (MBS), maleimido-benzoylsulfosuccinimide ester (sulfo-MBS), succinimidyl-4-(N-maleimidomethyl) cyclohexane-1-carboxylate (SMCC), succinimidyl-4-(p-meleimido-phenyl)butyrate (SMPP), sulfosuccinimidyl-4-(N-maleimidomethyl)cyclohexane-1-carboxylate (sulfo-SMCC) and sulfosuccinimidyl-4-(p-maleimidophenyl) butyrate (sulfo-SMPP).

In place of the maleimide-N-hydroxysuccinimide ester reactant, there may be employed the corresponding p-nitrophenyl esters or the 1-hydroxy-2-nitrobenzene sulfonic acid sodium salt esters, corresponding to the formula:

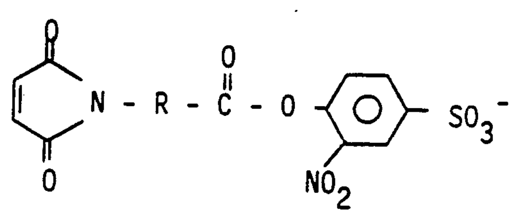

Upon reaction with an amino group of the protein, the following reaction occurs:

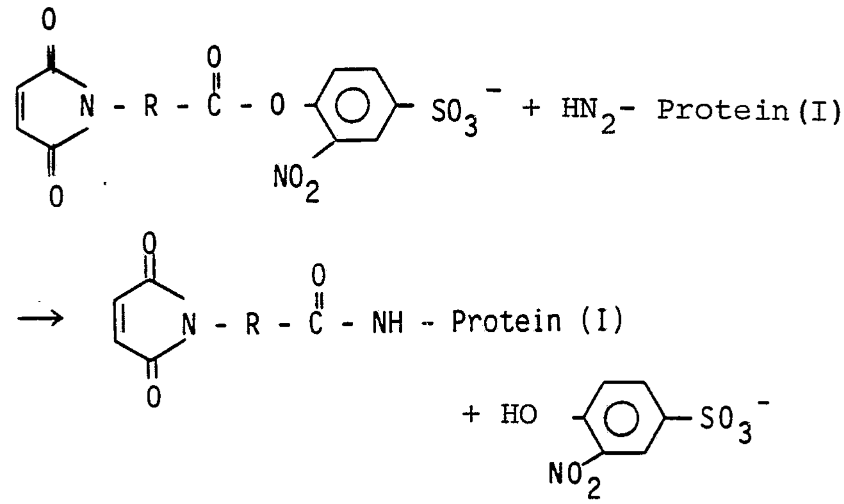

The proteins which are conjugated to form the conjugates in the present invention only rarely contain free thiol (sulfhydryl) groups. If such thio groups are present, thiolation is not required. Usually, however, it is necessary to introduce a thiol group using a thiolating agent. Any convenient thiolating agent may be employed, including N-succinimidyl 3-(2-pyridyldithio) propionate (SPDP), methyl 4-mercaptobutyrimidate in the presence of 4,4′-dithiopyridine, S-acetylmercaptosuccinic anhydride (SAMSA) or, preferably, N-succinimidyl-S-acetylthioacetate (SATA).

When the latter reagent is employed, the SATA reacts with protein amino groups to yield acetylthio derivatives, which, upon treatment with hydroxylamine, liberates free sulfhydryl groups. The modification of proteins using SATA and the subsequent hydroxylamine treatment are illustrated by the following equations:

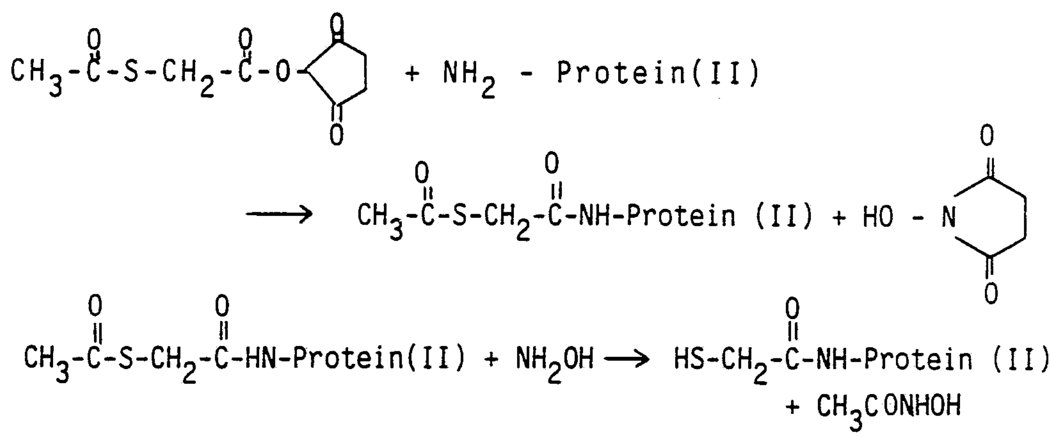

SATA is preferred over other potential thiolating agents, such as SPDP and SAMSA, since the addition and subsequent scrupulous removal of an excess of another thiol before completion of the conjugation is not required, in contrast to SPDP, and only one type of derivative can be formed, in contrast to SAMSA which can react at either of its anhydride carbonyls to produce mixed products.

The number of thiol groups introduced to the viral protein antigen and forming covalent links may vary widely from about 3.5 to about 11 per molecule of viral protein antigen. The number of thiol reactive groups formed on the immunogenic protein also may vary widely from about 5 to about 15.0 per molecule of highly immunogenic protein. The number of third reactive groups employed in any particular instance depends on a number of factors, including molecular weight, conformation and amino acid composition of the proteins in the reaction.

The invention is illustrated further by the following Examples:

Example 1

This Example illustrates the preparation of a conjugate influenza vaccine from influenza type A virus.

Purified diphtheria toxoid (D) was reacted with maleimido-caproic acid-N-hydroxysuccinimide ester (MCS) in the mole ratio of 1:1 at 20 to 25°C with stirring. The MCS was added in four equal portions to the DT over a period of 60 minutes and reaction was continued for a further 15 minutes after the final addition. The reaction mixture then was passed through a chromatography column packed with Bio-Gel P-6 DG to remove excess unreacted MCS. The column was previously equilibrated with a buffer solution of 0.1 M sodium citrate, 0.5% EDTA, pH 6.0 and was eluted with the buffer solution.

Purified hemagglutinin (HA) from an influenza type-A (Chile) virus, rendered soluble by bromelain cleavage of the formalin inactivated whole virus was thiolated by reaction with N-succinimidyl-S-acetylthioacetic acid (SATA) in the mole ratio of 1:1 at 20° to 25°C with stirring. The SATA was added in one batch to the HA and reaction was effected for 20 minutes. The reaction mixture then was passed through a chromatography column packed with Bio-Gel P-6 DG to remove excess unreacted SATA. The column was previously equilibrated with a buffer solution of 0.1 M sodium phosphate, 0.1 M sodium chloride, 0.05% sodium azide, pH 7.5 and eluted with that buffer solution.

The HA-AT product eluted from the chromatography column was deacetylated by reaction with hydroxylamine and excess unreacted hydroxylamine was removed on a chromatography column packed Bio-Gel P-6 DG which had previously been equilibrated with a buffer solution of 0.1 M sodium citrate, 0.5% EDTA, pH 6.0. The column was eluted with the same buffer solution.

The D-MCS product and the deacetylated HA-AT product obtained as described above were then reacted in a 1:1 mole ratio at 20° to 25°C with stirring for 18 to 24 hours. The reaction was terminated by the addition of an excess quantity of cysteine.HCl, followed by two further hours reaction with stirring at 20° to 25°C.

The reaction mixture was purified by application to a chromatography column packed with Sepharose CL-6B which had previously been equilibrated with a 0.06M phosphate buffered saline, pH 7.2 and 1:10,000 thimerosal. The product was filtered and concentrated.

The concentrate was diluted with additional quantities of the thimerosal buffer to vaccine strength, filtered and stored at 2° to 8°C for use. The protein concentration of the resulting HA-D conjugate vaccine was about 50 µg/ml and the HA content was about 30 µg HA/ml.

Example 2

This Example illustrates the preparation of a conjugate influenza vaccine from influenza type B virus.

The procedure of Example 1 was repeated, except that the hemagglutinin from an influenza type-B virus (USSR), was substituted for the type-A hemagglutinin. The resulting HA-D conjugate, when formulated at vaccine strength, had a protein content of about 50 µg/ml and an HA concentration of about 30 µg HA/ml.

Example 3

This Example illustrates the effectiveness of the HA-D vaccine produced by the procedure of Example 1 by two serologic assays, hemagglutination inhibition (HAI) and enzyme-linked immuno absorbent assay (ELISA).

Two HAI parameters are associated with a protective response to influenza vaccination: 1) achieving a post-vaccination HAI titer $\geq$ 40 and 2) achieving a post-vaccination HAI titer $\geq$ 4 times the pre-vaccination titer. By both of these parameters, the HA-D conjugate vaccine recipients had better responses than did recipients of the subunit vaccine or did the control group which was not vaccinated.

The results obtained are summarized in the following Table I:

Table I

| HAI Values | HA-D n=25 | Subunit n=25 | Control n=25 |
|---|---|---|---|
| % pre < 10 | 64 | 60 | 80 |
| % pre ≥ 40 | 0 | 4 | 4 |
| % post 1 ≥ 40* | 68 | 36 | 8 |
| % post 2 ≥ 40* | 80 | 44 | 8 |
| % post 1 ≥ 4X pre | 72 | 52 | 0 |
| % post 2 ≥ 4X pre | 72 | 52 | 0 |
| % pre < 10 with post 1 or 2 ≥ 4X pre | 75 | 73 | 0 |

* = statistically significant difference p 0.05

The persistence of protective levels of antibody measure by HAI six months post-vaccination is seen in a higher percentage of HA-D conjugate vaccine recipients than is seen in the subunit recipient group, as set forth in the following Table II:

Table II

| HAI Values | HA-D n=25 | Subunit n=25 | Control n=25 |
|---|---|---|---|
| % post 1 or 2 ≥ 40 and post 6 ≥ 40 | 95 | 70 | 0 |
| % post 1 or 2 ≥ 4X pre and post 6 ≥ 4X pre | 89 | 86 | 0 |
| % pre < 10, 40 post 1 or 2 and post 6 ≥ 40 | 92 | 73 | 0 |
| % pre < 10, 4X pre post 1 or 2 and post 6 ≥ 4X pre | 93 | 86 | 0 |

By ELISA testing using hemagglutinin presented as detergent extract (split) or as purified HA bound to the solid phase, the following results were generated. These results suggest an enhanced response to cognate (A/Chile) HA in either presentation by the HA-D cognate vaccine recipients relative to the response of the split vaccine recipients. These data also show enhanced reactivity in the conjugate vaccine recipients with respect to recognition of other HINI strain (A/Brazil) HA. The results are set forth in the following Table III:

Table III

FOLD RESPONSE TO FIRST DOSE

| VACCINE GROUP | A/Chile split | A/Chile HA | A/Brazil HA | A/Phil split | A/Phil HA | B/USSR HA |
|---|---|---|---|---|---|---|
| HA-D | 2.8 | 5.5 | 6.1 | 1.4 | 2.0 | 1.3 |
| SPLIT | 2.6 | 1.6 | 2.1 | 1.1 | 1.2 | 0.8 |

Example 4

Example 4

This Example illustrates the effectiveness of the HA-D vaccine produced by the procedure of Example 1 by measurement of cell-mediated responsiveness with results expressed as stimulation indices.

The absolute stimulation indices of each vaccine group at six months post-vaccination are presented in the following Table IV:

Table IV

STIMULATION INDICES FROM HA(A/Chile)-D CLINICAL TRIAL

| VACCINE | Rank Order of Stimulation Index[1] post 1 | post 2 | S.I.[2] post 6 |
|---|---|---|---|
| HA-D | 10/10 | 12/12 | average:7.8 |
| HA-D | 5/10 | 1/12 | |
| HA-D | 2/10 | 2/12 | |
| HA-D | 1/10 | 4/12 | |
| HA-D | 4/10 | 3/12 | |
| Subunit | 6/10 | 5/12 | average:5.0 |
| Subunit | 7/10 | 5/12 | |
| Subunit | 9/10 | 2/12 | |
| Subunit | 4/10 | 10/12 | |
| Subunit | 8/10 | 11/12 | |
| Subunit | 4/10 | 11/12 | |
| Subunit | 2/10 | 7/12 | |
| Control | 6/10 | 10/12 | |
| Control | 9/10 | 8/12 | |
| Control | 7/10 | 6/12 | |
| Control | 6/10 | 7/12 | |
| Control | 3/10 | 9/12 | |

[1] Rank ordering system where 1/10 or 1/12 is the highest or best response in each group and where 10/10 or 12/12 is the lowest or poorest response in each group

[2] Stimulation Index

$$\frac{\text{cpm } ^3\text{H-dTR incorporated in the presence of antigen}}{\text{cpm } ^3\text{H-dTR incorporated in the absence of antigen}}$$

The post-six month data presented above suggests persistance of cell-mediated immunity at a higher level in the group of subjects receiving HA-D conjugate vaccine relative to the group receiving the subunit vaccine.

Example 5

This Example illustrates the effectiveness of the vaccine produced by the procedure of Example 2.

Absolute stimulation index (S.I.) values for individual subjects and average stimulation indices for the two vaccine groups are presented in Table V below:

Table V

STIMULATION INDICES FROM HA(B/USSR)-D CLINICAL TRIAL

| VACCINE | Antigen: HA post 1 | HA post 2 | SPLIT post 1 | SPLIT post 2 | sum of HA and SPLIT post 1 | sum of HA and SPLIT post 2 |
|---|---|---|---|---|---|---|
| HA-D | 4.5 | 5.4 | 2.5 | 2.6 | 3.5 | 4.0 |
| HA-D | 6.7 | 8.6 | 1.6 | 4.3 | 4.2 | 6.5 |
| HA-D | 3.7 | 3.5 | 5.2 | 1.6 | 4.5 | 2.6 |
| HA-D | 10.7 | 1.5 | 4.9 | 1.2 | 7.8 | 1.4 |
| HA-D | 6.0 | 9.1 | 1.5 | 2.2 | 3.8 | 5.7 |
| HA-D | 13.5 | 4.3 | 1.5 | 1.5 | 7.5 | 2.9 |
| split | 1.7 | 9.7 | 3.3 | 3.3 | 2.5 | 6.5 |
| split | 2.6 | 2.3 | 0.9 | 2.6 | 1.7 | 2.4 |
| split | 2.6 | 4.4 | 3.2 | 1.2 | 2.9 | 2.8 |
| split | 1.8 | 3.1 | 2.0 | 1.3 | 1.8 | 2.2 |
| split | 2.0 | 2.8 | 1.4 | 1.0 | 1.7 | 1.9 |
| split | 1.9 | 2.4 | 1.8 | 2.3 | 1.8 | 2.3 |
| none | 1.3 | 4.9 | 0.8 | 1.6 | 1.0 | 3.2 |
| none | 1.2 | 2.7 | 0.7 | 1.1 | 0.9 | 1.9 |
| none | 1.0 | 1.5 | 0.7 | 0.8 | 0.8 | 1.2 |
| none | 0.9 | 1.0 | 1.6 | 0.6 | 1.2 | 0.8 |
| none | 1.3 | 4.3 | 0.9 | 1.5 | 1.1 | 2.9 |
| none | 1.0 | na | 0.6 | na | 0.8 | na |

na: not available

Average SI

| | | | | | | |
|---|---|---|---|---|---|---|
| HA-D | 7.5 | 5.4 | 2.9 | 2.2 | 5.3 | 3.8 |
| split | 2.1 | 4.1 | 2.1 | 2.0 | 2.1 | 3.0 |
| none | 1.1 | 2.9 | 0.9 | 1.1 | 1.0 | 2.0 |

Results suggest much higher S.I. values in the HA-D conjugate recipient group than in the split vaccine recipient group. This trend is observed at both post 1 and post 2 dates and in both antigen presentations: purified HA and split HA.

Example 6

This Example also illustrates the effectiveness of the vaccine produced by the procedure of Example 2.

Serologic response data was generated to the conjugate vaccine of Example 2 and to a conventional influenza subunit vaccine measured as geometric mean hemagglutination inhibition titers. The results are reproduced in the following Table VI:

Table VI

| | Vaccine | |
|---|---|---|
| | Conjugate | Subunit |
| pre-vaccination | 39.2 | 29.6 |
| 1 month post-vaccination | 287.6 | 225.2 |
| 2 months post-vaccination | 176.8 | 140.0 |

As may be seen from this Table VI, the vaccine of the invention elicits a greater immune response than the subunit vaccine.

In summary of this disclosure, the present invention provides a novel conjugate in which a weakly-immunogenic viral protein antigen is covalently-bonded to a strongly immunogenic protein to produce an enhanced immune response to the normally weakly-immunogenic antigen. Modifications are possible within the scope of the invention.

## Claims

1. A conjugate, characterized by a normally weakly-immunogenic viral protein antigen covalently bonded to a strongly immunogenic protein.

2. The conjugate claimed in claim 1, characterized in that said viral protein antigen is selected from the group consisting of influenza virus, hemagglutinin glycoprotein, rabies virus glycoprotein g, polio virus glycoproteins and rotavirus glycoproteins.

3. The conjugate claimed in claim 1, characterized in that said viral protein antigen is the hemagglutinin glycoprotein of the influenza virus.

4. The conjugate claimed in any one of claims 1 to 3, characterized in that said strongly immunogenic protein is derived from a bacterial organism.

5. The conjugate claimed in claim 4, characterized in that said strongly immunogenic protein is diphtheria toxoid or tetanus toxoid.

6. The conjugate claimed in any one of claims 1 to 5, characterized in that said proteins are covalently linked by a moiety joined to one of the proteins by an amide group and to the other of the proteins by a thioether group.

7. The conjugate claimed in claim 6, characterized in that said moiety has the structure:

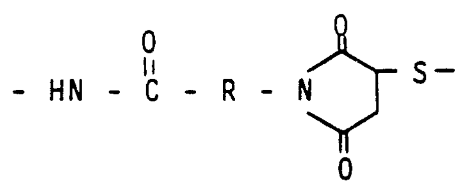

in which R is an optionally substituted phenylene group, cycloalkylene group or alkylene group containing from 1 to 12 carbon atoms.

8. The conjugate claimed in claim 7, characterized in that R is an alkylene group containing from 5 to 12 carbon atoms.

9. The conjugate claimed in any one of claims 6 to 8, characterized in that said thioether group results from reaction between sulfhydryl groups introduced to said weakly-immunogenic viral protein antigen and thiol reactive groups on the moiety joined through said amide groups resulting from reaction with amino groups on said strongly-immunogenic protein.

10. The conjugate claimed in claim 9, characterized in that about 3.5 to about 11 sulfhydryl groups are introduced per molecule of viral protein antigen and about 5 to about 15 thiol-reactive groups are present per molecule of highly immunogenic protein.